# EUROPÄISCHE PATENTANMELDUNG

(11) **EP 3 461 408 A1**
(43) Veröffentlichungstag der Anmeldung: **03.04.2019**
(21) Anmeldenummer: 17193747.7
(22) Anmeldetag: 28.09.2017
(51) Int. Cl.: A61B 6/00, A61B 46/10

(54) **BEDECKEN EINER ÄUSSEREN GEHÄUSEFLÄCHE WENIGSTENS EINER KOMPONENTE EINER ANLAGE, INSBESONDERE EINER BILDGEBUNGSANLAGE UND/ODER EINER ROBOTEREINHEIT**

(71) Anmelder: Siemens Healthcare GmbH, 91052 Erlangen (DE)
(72) Erfinder: Wiets, Michael, 91094 Langensendelbach (DE); Roehrig, Timo, 90429 Nürnberg (DE)

(57) **Zusammenfassung**

Die Erfindung betrifft ein Verfahren zum Bedecken einer äußeren Gehäusefläche wenigstens einer Komponente einer Anlage mit einem sterilen Hüllelement, umfassend die Schritte
- Erstes Verstellen der Anlage derart, dass die wenigstens eine Komponente eine Startposition einnimmt,
- Positionieren des sterilen Hüllelements in einer Ausgangsposition relativ zur Startposition der wenigstens einen Komponente der Anlage,
- Zweites Verstellen der Anlage derart, dass sich die wenigstens eine Komponente der Anlage entlang eines vorab bestimmten Verstellwegs in das Hüllelement hinein bewegt, sodass das Hüllelement die äußere Gehäusefläche der Komponente wenigstens teilweise bedeckt.

Die Erfindung betrifft ferner eine Anlage, insbesondere eine medizinische Bildgebungsanlage, eine Recheneinheit, ein Computerprogramm sowie computerlesbaren Datenträger.

## Beschreibung

Die Erfindung betrifft ein Verfahren zum Bedecken einer äußeren Gehäusefläche einer Komponente einer Bildgebungsanlage, eine entsprechende Recheneinheit, entsprechendes Computerprogramm, entsprechenden Datenträger sowie eine entsprechende Bildgebungsanlage.

Die intraoperative Bildgebung, bei der während eines medizinischen Eingriffs der Verlauf bzw. Ergebnisse des Eingriffs direkt bildlich dargestellt, kontrolliert und ggf. korrigiert werden können, ist auf dem Vormarsch. Deutschlandweit wird derzeit bereits in über zweihundert Krankenhäusern mit intraoperativer Bildgebung gearbeitet. Bekannt sind Operationen unter Einsatz der klassischen Röntgenbildgebung mit einem C-Bogen-Röntgengerät, des intraoperativen Ultraschalls, sowie die neueren Anwendungen der intraoperativen Computertomographie, der intraoperativen Magnet-Resonanz-Tomographie und der intraoperativen Angiographie oder Kombinationen daraus.

Drei wesentliche Vorteile machen die intraoperative Bildgebung so attraktiv. Einerseits eröffnet die Anwendung der medizinischen Bildgebung während einer Operation für eine Vielzahl von Patienten, für die wegen der Art ihrer Erkrankung das konventionelle Operationsverfahren nicht in Frage kommt, eine neue Therapieoption. Zudem sind Eingriffe unter Anwendung von intraoperativer Bildgebung in aller Regel minimalinvasiv oder zumindest weniger invasiv als die konventionelle Operation und damit Patienten-schonend. Dies liegt daran, dass die medizinische Bildgebung Aufschluss über eine anatomische Situation liefert, die nun nicht mehr direkt, also am oder im Patienten durch optische Einsicht oder Ertasten ermittelt werden muss. Nicht zuletzt verbessert die intraoperative Bildgebung die Qualität des Behandlungsergebnisses, da während des Eingriffs Kontrollbilder entstehen und Komplikationen vermieden werden können. Vor Ort kann das Operationsergebnis dargestellt und überprüft werden, wie zum Beispiel die korrekte Positionierung eines Implantats, beispielsweise einer Aortenklappe, Pedikel-Schrauben oder einer Frakturversorgung. Die Anzahl notwendiger Revisionsoperationen wird reduziert, zum Nutzen der Patienten und im Sinne eines kosteneffizienten Gesundheitswesens.

Durch den Einsatz der intraoperativen Bildgebung ergeben sich erhebliche Veränderungen. Operationsabläufe und damit auch deren Strukturierung bzw. Planung ändern sich grundlegend. Das neue Werkzeug erfordert auch Veränderungen der Ausbildung und des Trainings des gesamten Operationspersonals.

Eine solche Veränderung betrifft auch die Vorbereitung der zum Einsatz kommenden Bildgebungsanlage für den Einsatz in einer sterilen Umgebung. Vor einem medizinischen Eingriff werden involvierte Körperregionen des Patienten, Operationsinstrumente, Werkzeuge, Operationsmaterial, Geräte zur Überwachung der Vitalfunktionen des Patienten oder Beatmungsgeräte oder dergleichen sterilisiert bzw. desinfiziert. Auch das Operationspersonal unterzieht sich vor einem Eingriff standardisierter Desinfektionsprozeduren und trägt entsprechende Schutzbekleidung wie Kittel, Haube, Handschuhe, Mundschutz, usw..

Eine medizinische Bildgebungsanlage, die während eines Eingriffs eingesetzt werden soll, muss ebenfalls für das sterile Arbeiten ausgelegt sein. Insbesondere von Teilen der Anlage, die dicht beim Patienten bzw. über ihm positioniert sind, dürfen keine Keime oder Partikel wie Schmutz oder Staub ausgehen. Umgekehrt ist es von Vorteil, wenn die Bildgebungsanlage bei der intra-operativen Bildmessung nicht übermäßig verschmutzt wird, beispielsweise durch Körperflüssigkeiten des operierten Patienten, die bei einem Eingriff frei gesetzt werden können, da eine Reinigung der Bildgebungsanlage aufwändig und damit zeitintensiv wäre.

Entsprechend werden heutzutage sterile Tücher oder Folien zum Abdecken der Bildgebungsanlage verwendet. Diese werden vor einem Eingriff durch das Operationspersonal um die Anlagenteile gelegt, gezogen oder gewickelt und mit Gummizügen, elastischen Bändern, Klebe- und/oder Klettstreifen befestigt. Oft, insbesondere aus wirtschaftlichen Gründen, weisen die Tücher eine nicht an die Bildgebungsanlage angepasste Form oder Größe auf. Zudem können mehrere Tücher notwendig sein, um die Anlage ausreichend zu bedecken, was beim Bedienpersonal ein hohes Maß an Erfahrung und Kenntnis voraussetzt, in welcher Reihenfolge und wie die Tücher jeweils angebracht bzw. wie unter Umständen benachbarte Tücher mit einander verbunden oder überlagert werden müssen. Darüber hinaus sind die sterilen Tücher oft so groß dimensioniert und unhandlich, dass mehrere Personen zum Fixieren bzw. (Vor-)Positionieren erforderlich sind. Alles in allem ist das sterile Einhüllen der Bildgebungsanlage ein personal- und zeitintensiver Vorbereitungsschritt.

Ausgehend davon ist es eine Aufgabe der vorliegenden Erfindung, Alternativen zum Bedecken einer äußeren Gehäusefläche wenigstens einer Komponente einer intraoperativ eingesetzten Anlage, insbesondere einer intraoperativ eingesetzten Bildgebungsanlage bereit zu stellen, welche das Einhüllen maßgeblich vereinfachen und beschleunigen und somit die beschriebenen Nachteile überwinden. Insbesondere ist es Aufgabe der Erfindung, unerfahrenem Bedienpersonal das fachgerechte Einhüllen zu ermöglichen.

Diese Aufgabe wird gelöst durch Verfahren zum Bedecken einer äußeren Gehäusefläche wenigstens einer Komponente einer Anlage, insbesondere einer Bildgebungsanlage, mit einem sterilen Hüllelement, durch entsprechende Recheneinheit und Anlage, durch entsprechendes Computerprogramm und computerlesbaren Datenträger gemäß den unabhängigen Ansprüchen. Bevorzugte und/oder alternative, vorteilhafte Ausgestaltungsvarianten sind Gegenstand der abhängigen Ansprüche.

Nachstehend wird die erfindungsgemäße Lösung der Aufgabe in Bezug auf das beanspruchte Verfahren als auch in Bezug auf die beanspruchten Vorrichtungen beschrieben. Hierbei erwähnte Merkmale, Vorteile oder alternative Ausführungsformen sind ebenso auch auf die anderen beanspruchten Gegenstände zu übertragen und umgekehrt. Mit anderen Worten können gegenständliche Ansprüche (die beispielsweise auf ein Verfahren gerichtet sind) auch mit Merkmalen, die in Zusammenhang mit einer der Vorrichtungen beschrieben oder beansprucht sind, weitergebildet sein. Die entsprechenden funktionalen Merkmale des Verfahrens werden dabei durch entsprechende gegenständliche Module oder Einheiten ausgebildet.

Die vorliegende Erfindung betrifft in einem ersten Aspekt ein Verfahren zum Bedecken einer äußeren Gehäusefläche wenigstens einer Komponente einer Anlage, insbesondere einer Bildgebungsanlage, mit einem sterilen Hüllelement. Die Bildgebungsanlage ist insbesondere eingerichtet für eine Bildmessung eines in der Bildgebungsanlage befindlichen Untersuchungsobjektes. Das Verfahren umfasst eine Vielzahl von Schritten.

Die Erfindung betrifft in einem zweiten Aspekt eine Anlage, die insbesondere als medizinische Bildgebungsanlage ausgebildet sein kann, zum Bedecken einer äußeren Gehäusefläche wenigstens einer Komponente der Anlage mit einem sterilen Hüllelement. Die eingerichtet ist, das erfindungsgemäße Verfahren auszuführen.

Bei der Anlage kann es sich bevorzugt um eine medizinische Bildgebungsanlage handeln, besonders bevorzugt um ein C-Bogen-Röntgengerät. Die Bildgebungsanlage kann auch als klassisches Ultraschall-Gerät ausgebildet sein. Andere Ausgestaltungsvarianten der medizinischen Bildgebungsanlage sind ebenfalls denkbar und liegen im Rahmen der hiesigen Erfindung.

Die wenigstens eine Komponente der Bildgebungsanlage kann jeder/jede/jedes Teil, Teilbereich, Element, Einheit oder Modul der Bildgebungsanlage sein, welche von der Bildgebungsanlage umfasst ist, insbesondere kann die wenigstens eine Komponente zur Bilderfassung bzw. zur Bildmessung. Die Gesamtheit aller Komponenten der Bildgebungsanlage kann die gesamte Bildgebungsanlage einschließen. Alternativ und bevorzugt werden erfindungsgemäß jedenfalls die Komponenten der Bildgebungsanlage bedeckt, die einem Untersuchungsobjekt bei einer intraoperativen Bildmessung nahe kommen bzw. auf das Untersuchungsobjekt ausgerichtet sind.

In einem bevorzugten Beispiel der Erfindung ist die Bildgebungsanlage als C-Bogen-Röntgenanlage ausgebildet und die wenigstens eine Komponente ist eine aus der Gruppe der folgenden Komponenten: ein C-Bogen, eine Röntgenquelle, ein Generator, ein Detektor und ein Schlitten.

In einem anderen bevorzugten Ausführungsbeispiel der Erfindung ist die Anlage als intraoperative Robotereinheit ausgebildet. Die Robotereinheit ist eine in der Regel physikalisch von einer intraoperativ eingesetzten Bildgebungsanlage getrennte Robotereinheit. Derartige Robotereinheiten kommen beispielsweise zum ortsfesten Fixieren als intra-operative Bohrführung o.Ä. für einen Arzt zum Einsatz, insbesondere bei Operationen an Knochen. Mit anderen Worten wird eine Robotereinheit während eines Eingriffs dicht an ein Untersuchungsobjekt heran gefahren und fixiert, beispielsweise eine Bohrschablone in definierter Position relativ zum Untersuchungsobjekt. Insofern ist eine sterile Bedeckung auch dieser Robotereinheit bzw. wenigstens einzelner ihrer Komponenten wünschenswert. Die Robotereinheit kann mit der Bildgebungsanlage zumindest in Datenaustausch stehen. Selbstredend ist die Robotereinheit auch unabhängig von einer medizinischen Bildaufnahme während des medizinischen Eingriffs einsetzbar, also auch dann, wenn die Bildgebungsanlage gerade keine Bilddaten erfasst. Eine Komponente einer Robotereinheit kann beispielsweise ihre Haltevorrichtung zur Fixierung der oben angesprochenen Bohrschablone sein, ein beliebig großer (Teil)Abschnitt des um mehrere Raumachsen verstellbaren Ständers oder aber der Roboterfuß. Im Übrigen gelten dieselben Aussagen, die auch für die Komponenten der Bildgebungsanlage gemacht wurden, sofern übertragbar.

Eine Bildmessung ist im Rahmen der Erfindung als Erfassung von medizinischen Bilddaten zu verstehen, beispielsweise von Angiographie-Aufnahmen.

Bei einem Untersuchungsobjekt wird im Folgenden und ohne Beschränkung der Allgemeinheit von einem Patienten ausgegangen, wobei es sich meist um einen Menschen handelt. Grundsätzlich kann der Patient aber auch ein Tier sein. Im Folgenden werden daher die beiden Begriffe "Untersuchungsobjekt" und "Patient" auch synonym verwendet.

Im Rahmen dieser Erfindung beschreibt eine äußere Gehäusefläche einer Komponente einer Anlage einen Teil bzw. einen Teilbereich ihres äußeren Gehäuses, typischerweise auch als Cover bezeichnet, welches die Komponente der Anlage nach außen hin begrenzt. Die äußere Gehäusefläche kann das gesamte äußere Gehäuse der Komponente umfassen oder nur einen oder mehrere einzelne oder zusammenhängende Teile bzw. Teilbereiche davon.

Das Hüllelement ist erfindungsgemäß als wenigstens eine Folie oder als wenigstens ein Tuch aus einem Material, Gewebe oder Gewirk ausgestaltet, welches wenigstens die äußere Gehäusefläche der wenigstens einen Komponente bedeckt, die während des medizinischen Eingriffs und/oder der Bildmessung dem Untersuchungsobjekt zugewandt ist. Mit anderen Worten weist das Hüllelement wenigstens eine Fläche auf, die der Größe der äußeren Gehäusefläche entspricht. Als Materialien kommen alle Materialien in Frage, die flexibel sind, leicht und eine begrenzte Luftdurchlässigkeit haben, z.B. Kunststofffolien, Metallfolien, Textilien, beschichtetes Papier und Kombinationen davon. Das Hüllelement kann in Ausgestaltungen der Erfindung Gummizüge, Zugbänder, Klebe- und/oder Klettstreifen umfassen. Dies vereinfacht das sachgemäße Anbringen und Entfernen vor und nach einem medizinischen Eingriff und effektiviert somit den medizinischen Arbeitsprozess. Das Hüllelement kann flächig, also im Wesentlichen plan, Tütenförmig oder Schlauchförmig ausgebildet sein. Mit anderen Worten kann das Hüllelement auch an allen bis auf eine oder zwei sich gegenüberliegenden Seiten geschlossen ausgebildet sein. Ein derartiges Hüllelement wird einer Komponente übergezogen, übergestülpt oder das Hüllelement wird über die Komponente abgerollt. Die Größe eines Hüllelements ist beliebig und frei wählbar, je nach einzuhüllender Komponente. Jede dieser Hüllelement-Ausgestaltungen kann einer an die Form und/oder Größe der Komponente bestmöglich angepassten Form bzw. Größe entsprechen. Es können mehrere Hüllelemente vorgesehen sein, die zusammen wenigstens eine oder mehrere Komponenten der Anlage einhüllen. Alternativ kann ein Hüllelement derart ausgestaltet sein, dass es mehrere Komponenten einhüllen kann.

Das Hüllelement weist ferner eine sterile Außenseite auf, die der äußeren Gehäusefläche der Komponente abgewandt ist, wenn das Hüllelement an der äußeren Gehäusefläche angeordnet ist. Derart wird der Anforderung nach einer sterilen Umgebung während eines medizinischen Eingriffs besonders Rechnung getragen. Durch dieses Hüllelement wird gewährleistet, dass Verunreinigungen wie Staub oder Schmutzpartikel oder infektiöse Keime, die sich am Gehäuse der Bildgebungsmodalität befinden, zum Beispiel in schlecht zugänglichen Gehäuse-Spalten oder Gelenk-Hüllen, die bei einer Bewegung einer Komponente, beispielsweise einer Bildmessung verschoben, rotiert, gekippt oder dergleichen werden, zurückgehalten werden. Zudem wird eine Gefährdung des Untersuchungsobjektes durch das Hüllelement selbst verhindert. Das Hüllelement kann insbesondere in Form eines Einwegproduktes ausgestaltet sein, welches erst kurz vor dem Gebrauch durch Operationspersonal aus einer sterilen Verpackung entnommen und erfindungsgemäß an seine finale Position während der Bildmessung verbracht wird. Umgekehrt schützt ein steriles Hüllelement gleichermaßen die Anlage vor Kontamination mit Körperflüssigkeiten oder infektiösen Keimen für den Fall, dass das Untersuchungsobjekt an einer ansteckenden Krankheit, wie zum Beispiel bakteriellen oder viralen Infektionen leidet. Damit eignet sich die Erfindung besonders für intraoperative Bildmessungen.

In einem ersten Schritt des erfindungsgemäßen Verfahrens wird die Anlage derart verstellt, dass die wenigstens eine Komponente, die durch das Hüllelement bedeckt, abgedeckt, verpackt oder eingehüllt werden soll, eine Startposition einnimmt. Dieser erste Verstellschritt erfolgt bevorzugt automatisch, kann aber auch Nutzer-induziert ablaufen. Das Verstellen umfasst Bewegungen bzw. Verschiebungen, also Translation, Rotation, Torsion, Verkippungen, Verschwenkungen oder dergleichen der gesamten Anlage oder einzelner Teile oder Module, insbesondere einzelner Komponenten, derart, dass die wenigstens eine einzuhüllende Komponente eine Startposition einnimmt. Mit anderen Worten wird die einzuhüllende Komponente durch eine Bewegung der Anlage und/oder durch eine Bewegung der wenigstens einen Komponente selbst in die Startposition verfahren, geführt oder bewegt. Die Startposition, vorab für die wenigstens eine Komponente abrufbar hinterlegt, also abgespeichert, insbesondere in Form von Koordinaten aller Raumrichtungen in Bezug zu einem Referenz-Koordinatensystem, stellt den Ausgangspunkt für eine weitere erfindungsgemäße Verstellbewegung dar, wie sie weiter unten beschrieben wird. Die Startposition der Komponente kann gekennzeichnet sein durch eine gute Erreichbarkeit für Bedienpersonal. Mit anderen Worten ist die wenigstens eine Komponente in der Startposition für einen Nutzer händisch gut zu erreichen, beispielsweise kann der Nutzer die Komponente in der Startposition mit beiden Armen leicht umgreifen. Insbesondere befindet sich die Startposition in einer Höhe relativ zum Fußboden, die einer Becken- oder Schulterhöhe eines Nutzers, beispielsweise in einer Höhe zwischen 90 und 160 cm entspricht, sodass dieser in aufrechter oder leicht gebückter Haltung die Komponente erreichen kann. Die Startposition der Komponente kann auch eine spezielle Orientierung bzw. Anordnung der Komponente umfassen, die die Erreichbarkeit für den Nutzer verbessert.

In einem zweiten Verfahrensschritt erfolgt ein Positionieren des sterilen Hüllelements in einer Ausgangsposition relativ zur Startposition der wenigstens einen Komponente der Anlage. Mit anderen Worten ergibt sich die Ausgangsposition des Hüllelements infolge der Definition der Startposition der wenigstens einen Komponente. Die Ausgangsposition befindet sich in räumlicher Nähe zu der Komponente in der Startposition, beispielsweise weist die Ausgangsposition einen Abstand von 5cm bis 25cm zur äußeren Gehäusefläche der Komponente in der Startposition auf. Das Positionieren des Hüllelements in der Ausgangsposition kann auch ein Öffnen, Auffalten, Aufrollen, Aufklappen oder allgemeiner Anordnen des Hüllelements umfassen, insbesondere dann, wenn das Hüllelement Tüten- oder schlauchförmig ausgebildet ist. Derart wird schon in der Ausgangsposition für das Hüllelement erreicht, dass es mit seiner Innenseite bzw. Innenfläche der äußeren Gehäusefläche der Komponente zugewandt ist. Dies ermöglicht bzw. erleichtert eine korrekte Anlagerung bzw. Bedeckung der äußeren Gehäusefläche der Komponente durch den sich anschließenden weiteren, zweiten Verstellschritt, dem dritten Schritt des erfindungsgemäßen Verfahrens.

Gemäß einem bevorzugten Beispiel der Erfindung erfolgt das Positionieren des Hüllelements in der Ausgangsposition durch einen Nutzer. Dieser hält bevorzugt auch während des dritten Verfahrensschritts das Hüllelement zumindest teilweise in der Ausgangsposition fest. In einer anderen Ausgestaltung der Erfindung umfasst das Positionieren des Hüllelements in der Ausgangsposition ein Befestigen des Hüllelements in der Ausgangsposition. Dazu können an der wenigstens einen Komponente und/oder am Hüllelement selbst entsprechende Befestigungsmittel wie beispielsweise Halteklammern, Klettverschlüsse, Klebestreifen oder dergleichen vorgesehen sein. Derart kann vorteilhaft der folgende Schritt auch ohne Benutzer-Aktion erfolgen.

Dieser dritte Verfahrensschritt sieht vor, dass die Anlage bzw. die wenigstens eine Komponente derart verstellt wird, dass sich die wenigstens eine Komponente der Anlage entlang eines vorab bestimmten Verstellwegs in das Hüllelement hinein bewegt, sodass das Hüllelement die äußere Gehäusefläche der Komponente wenigstens teilweise bedeckt. Dieser Schritt startet bevorzugt automatisch, beispielsweise auf Nutzereingabe hin oder nach Detektion, dass das Hüllelement in der Ausgangsposition angeordnet wurde. Der Verstellweg entspricht eine Trajektorie im Raum, bevorzugt mit Bezug zu einem Referenzkoordinatensystem der Anlage, entlang welcher die Komponente durch oben schon aufgezählte Bewegungen verstellt wird, sodass beispielsweise der Nutzer, der das Hüllelement in der Ausgangsposition hält, weder seine Position noch seine Haltung anpassen muss. Durch das zweite Verstellen der Komponente wird diese in das Hüllelement hineinbewegt. Mit anderen Worten wird die Komponente auf ihrem Verstellweg durch die Ausgangsposition des Hüllelements geführt. Derart wird das Hüllelement an die äußere Gehäusefläche der Komponente angelagert, sprich, die Komponente wird insbesondere bei tütenoder schlauchförmigen Hüllelementen in dasselbe hineingefahren, wobei das Hüllelement zumindest teilweise mit der Komponente mitgeführt und ggf. abgerollt, entfaltet bzw. gezogen wird und dadurch ihre Gehäusefläche einhüllt. Im Falle eines flächigen Hüllelements wird dieses ebenfalls zumindest teilweise von der Komponente mitgeführt, wodurch ebenfalls die Gehäusefläche bedeckt wird. Das Hüllelement selbst verbleibt während des zweiten Verstellen der Anlage zumindest teilweise in seiner Ausgangsposition, entweder weil es durch den Nutzer oder die Befestigungsmittel dort fixiert wird.

Zusammenfassend vereinfacht und beschleunigt die erfindungsgemäße Vorgehensweise den Vorbereitungsprozess für einen operativen Eingriff und insbesondere für eine intraoperative Bildmessung, da nunmehr lediglich das Hüllelement in eine initiale Position, die Ausgangsposition, gebracht werden und dort ggf. vom Nutzer gehalten werden muss. Beim zweiten Verstellen der Anlage kann der Nutzer vorteilhaft in seiner Position und Haltung verbleiben bzw. erübrigt oder minimiert sich eine Nutzer-Beteiligung. Lediglich muss er ggf. noch händisch ein korrektes Bedecken der äußeren Gehäusefläche durch das Hüllelement kontrollieren bzw. bewirken, indem er das Hüllelement entsprecht mit kleinen Handbewegungen nachführt, abrollt, entfaltet und/oder seine Position korrigiert.

Gemäß einer Ausgestaltungsvariante erfolgt das zweite Verstellen der Anlage kontinuierlich, bis die wenigstens eine Komponente eine vorab definierte Endposition eingenommen hat. Die kontinuierliche Bewegung erfolgt also unterbrechungsfrei bis in eine Endposition hinein. Die Endposition ist dabei eine Position, in der das Hüllelement die äußere Gehäusefläche wie vorsehen bedeckt. Die Endposition kann darüber gekennzeichnet sein, dass das Hüllelement im Wesentlichen vollständig abgerollt, entfaltet oder geöffnet oder dergleichen ist, wenn die Komponente die Endposition erreicht hat. Das kontinuierliche Verstellen kann mit vorab festgelegter Geschwindigkeit erfolgen, die so gewählt wird, dass es dem Nutzer bequem möglich ist, während des Verstellens das Hüllelement abzurollen, zu entfalten, seine Position zu korrigieren oder dergleichen.

Gemäß einer alternativen Ausgestaltungsvariante erfolgt das zweite Verstellen der Anlage schrittweise zwischen Startposition und einer vorab definierten Zwischenposition, zwischen vorab definierten Zwischenpositionen und/oder zwischen vorab definierter Zwischenposition und einer vorab definierten Endposition, bis die wenigstens eine Komponente die vorab definierte Endposition eingenommen hat.

Bezüglich der hiesigen Endposition gilt das bereits Gesagte. Bei einer Zwischenposition handelt es sich erfindungsgemäß um eine vorab festgelegte Position der Komponente, beispielsweise wieder mit Bezug zum Referenzkoordinatensystem der Anlage, die entlang ihres Verstellweges zwischen Startposition und Endposition angeordnet ist. Es können eine oder eine Vielzahl von Zwischenpositionen vorgesehen sein, und zwar in Abhängigkeit der Größe und/oder Form der wenigstens einen Komponente. Je größer eine Komponente ist, beispielsweise der C-Bogen einer C-Bogen-Röntgenanlage, umso mehr Zwischenpositionen können vorgesehen sein. Das zweite Verstellen erfolgt in dieser Ausführung also intervall-artig, mit einem Zwischenstopp pro Zwischenposition. Jeder Zwischenstopp ermöglicht dem Bedienpersonal, eine Kontrolle bzw. eine Nachbesserung der Anordnung des Hüllelements bezüglich der äußeren Gehäusefläche der Komponente vorzunehmen, ohne dass gleichzeitig die Verstellbewegung fortgeführt wird. Dies ist beispielsweise vorteilhaft, bei einer unebenen Gehäuseoberfläche der Komponente, die beispielsweise durch Auskragungen oder Spalten ein unerwünschtes Mitführen des Hüllelements verursachen könnte. Die Schrittweite zwischen den Zwischenpositionen entlang des Verstellweges kann beispielsweise zwischen 30cm und 70cm liegen, aber auch insbesondere kleiner sein.

Gemäß einer bevorzugten Ausführungsvariante umfasst das erfindungsgemäße Verfahren auch folgenden Schritt:
- Prüfen zumindest für die Startposition, eine vorab definierte Zwischenposition und/oder die Endposition der Komponente, ob das Hüllelement die äußere Gehäusefläche der Komponente soweit bedeckt, wie es der jeweiligen Position der Komponente auf ihrem Verstellweg entspricht.

Erfindungsgemäß ist vorgesehen, wenigstens einmal im Rahmen des zweiten Verstellens eine korrekte Positionierung des Hüllelements zu überprüfen. Bevorzugt sind mehrere Prüfschritte umfasst, idealerweise für jede vorhandene bzw. definierte Position der Komponente. Derart stellt die Erfindung sicher, dass das zweite Verstellen fehlerfrei erfolgt, mit anderen Worten, dass die Komponente korrekt durch das Hüllelement bedeckt wird. Der Prüfschritt kann beispielsweise per Sichtkontrolle durch Bedienpersonal erfolgen. Alternativ und bevorzugt erfolgt der Prüfschritt automatisch durch die Anlage. Kombinationen von verschiedenen Prüfschritten für verschiedene Komponentenpositionen sind ebenfalls denkbar.

In einer besonders bevorzugten Ausgestaltungsvariante der Erfindung umfasst das Prüfen daher ein Erfassen, ob ein Kontakt zwischen einem die jeweilige Position der Komponente repräsentierenden ersten Kontaktelement an der Komponente und einem die jeweilige Position repräsentierenden zweiten Kontaktelement an dem Hüllelement geschlossen ist.

Entsprechend umfassen in einer anderen Ausgestaltungsvariante der Erfindung die wenigstens eine Komponente und das Hüllelement jeweils wenigstens ein Kontaktelement, die derart auf der Gehäuseoberfläche der Komponente bzw. der Oberfläche des Hüllelements angeordnet sind, dass ein Kontaktschluss zwischen denjenigen paarigen, eine jeweilige Position aus Startposition, vorab definierter Zwischenposition und/oder Endposition der Komponente repräsentierenden Kontaktelementen anzeigt, dass das Hüllelement die äußere Gehäusefläche der Komponente soweit bedeckt, wie es der jeweiligen Position der Komponente auf ihrem Verstellweg entspricht.

Mindestens ist ein Kontaktelement-Paar für eine der oben genannten Positionen der Komponente vorgesehen, wobei sich ein Kontaktelement jeweils auf der Gehäuseoberfläche der Komponente und eines auf der Hüllelement-Oberfläche befindet. Die Kontaktelemente sind so platziert, dass sie sich, sofern das Hüllelement in einer bestimmten Komponentenposition entlang des Verstellweges korrekt an der Gehäusefläche der Komponente anliegt, in einer räumlichen Nähe zu einander befinden, die ausreicht, einen Kontaktschluss zwischen beiden Kontaktelementen zu bewirken. Die Kontaktelemente können beispielsweise als an sich bekannte Reed-Kontakte oder RFID-Kontakte oder dergleichen ausgebildet sein. Entsprechend umfasst die Anlage in dieser Variante der Erfindung auch eine entsprechende Auslese- und Auswerte-Einheit, die insbesondere eingerichtet ist, einen Kontaktschluss für wenigstens eine der Komponentenpositionen zu detektieren und auszuwerten.

Sofern ein Kontakt zwischen zwei paarigen Kontaktelementen nicht schon alleine durch Erreichen der jeweiligen Position durch die Komponente geschlossen wird und die Prüfung einen mangelnden Kontaktschluss ergibt, hat der Nutzer nun Anreiz und Gelegenheit, den Kontakt durch eine händische Korrekturbewegung zu schließen und den korrekten Sitz des Hüllelements zu bewirken.

In einer weiteren Ausgestaltungsform der Erfindung weisen die Kontaktelemente der Komponente und des Hüllelements auf den jeweiligen Oberflächen einen Abstand zwischen 30cm und 70cm auf. Dieser Abstand entspricht in Anbetracht der gängigen Komponenten-Ausmaße von heutigen Bildgebungsanlagen einem guten Schrittmaß für die Prüfung, sodass ein korrekter Sitz des Hüllelements in allen Phasen des zweiten Verstellens sichergestellt ist, und zwar auch ohne dass der Nutzer seine Position und Haltung während des zweiten Verstellens ändern muss. In Bezug auf die Anlage in Form einer Robotereinheit kann das Abstandsmaß auch kleiner gewählt werden. Die Kontaktelemente sind nicht notwendigerweise entlang des Verstellwegs der Komponente angeordnet bzw. nicht notwendigerweise derart angeordnet, dass der Nutzer, der das Hüllelement während des zweiten Verstellens teilweise in der Ausgangsposition hält sie sehen kann. Insbesondere können Kontaktelemente in dem Nutzer abgewandten Gehäuseflächenbereichen angeordnet sein. Insofern wird insbesondere eine fehlerhafte bzw. unzureichende Sichtprüfung durch den Nutzer umgangen.

In Weiterbildung der Erfindung sieht eine Ausgestaltungsvariante vor, dass das zweite Verstellen der Anlage auf entsprechende Eingabe durch einen Nutzer hin gestartet und/oder gestoppt werden kann. Die Eingabe kann mittels Eingabe-Einheit der Anlage erfolgen, wobei Eingabeoptionen, die auf händische Betätigung verzichten, vorzuziehen sind. Insbesondere kommen eine Sprach-Eingabe oder eine Eingabe mittels Fußschalter oder dergleichen zum Einsatz, da so die Hände des Nutzers frei bleiben und zum Halten des Hüllelements zur Verfügung stehen.

Der Nutzer kann insbesondere eine kontinuierliche Verstellbewegung starten oder stoppen. Der Nutzer kann alternativ jeden Verstellschritt zwischen beliebigen Komponentenpositionen des schrittweisen zweiten Verstellens, starten oder stoppen. Ein Stoppen der Verstellbewegung kann beispielsweise dann notwendig sein, wenn das Hüllelement unerwünscht von der Komponente auf ihrem Verstellweg mitgeführt wird und dadurch beschädigt werden könnte oder der Nutzer beim Einhüllen der Komponente gestört wird. Ein Starten des schrittweisen Verstellens kann insbesondere in Abhängigkeit des Ergebnisses des Prüfschrittes für die aktuell eingenommene Komponentenposition erfolgen.

Entsprechend sieht eine weitere Ausgestaltungsvariante vor, dass ein weiteres, zweites Verstellen der Anlage von einer Position in eine entlang des Verstellwegs folgende Position der Komponente nur dann erfolgt, wenn ein vorheriger Prüfschritt ergibt, dass das Hüllelement für eine vorherige Position der Komponente die äußere Gehäusefläche der Komponente soweit bedeckt, wie es der vorherigen Position der Komponente auf ihrem Verstellweg entspricht und/oder der Nutzer dies per Eingabe über eine Eingabeeinheit der Bildgebungsanlage bestätigt, beispielsweise wieder mittels Fussschalter. Mit anderen Worten kann vorgesehen sein, dass das zweite Verstellen blockiert wird, solange der Prüfschritt für die vorherige Komponentenposition negativ ausfällt.

Gemäß einer anderen Ausgestaltungsvariante der Erfindung erfolgt das erste Verstellen der Anlage auf Aktivierung eines Hüll-Modus der Anlage hin automatisch. Im Hüll-Modus der Anlage in Form einer Bildgebungsanlage ist eine Bildmessung mit derselben ausgeschlossen. Der Hüll-Modus dient dazu, wenigstens eine Komponente der Anlage steril zu bedecken bzw. zu verpacken. Im Hüll-Modus greift die Anlage auf Prozess-Routinen des erfindungsgemäßen Verfahrens zu, beispielsweise ruft in diesem Modus hinterlegte Informationen bzgl. Startpositionen und/oder Verstellwegen und/oder Endpositionen von Komponenten etc. ab und aktiviert ihre Auslese- und AuswertEinheit zur Ausführung wenigstens eines Prüfschritts. Besonders vorteilhaft startet die Anlage automatisch auf Aktivierung des Hüllmodus das erste, erfindungsgemäße Verstellen der Anlage, sodass die wenigstens eine Komponente in die Startposition gebracht wird. Die wenigstens eine Komponente kann insbesondere eine Komponente der Anlage in Form einer Bildgebungsanlage sein, die in jedem Fall, also für jede Art von Bildmessung steril umhüllt werden muss, beispielsweise C-Bogen, Detektor und/oder Röntgenquelle eines C-Bogen-Röntgengeräts. Dabei kann die wenigstens eine Komponente auch unter Berücksichtigung einer notwendigen bzw. sinnvollen Reihenfolge von einzuhüllenden Komponenten ausgewählt werden. Alternativ und/oder zusätzlich kann ein Nutzer daher zuvor Eingaben zu der Komponente machen, die bei Aktivierung des Hüllmodus als erste verpackt werden soll.

Besonders bevorzugt ist der Hüll-Modus ein interaktiver Modus, sodass die Anlage in diesem Modus Eingaben eines Nutzers im Sinne des erfindungsgemäßen Verfahrens, beispielsweise Eingaben zur Bestätigung eines positiven Prüfschritts, Eingaben bzgl. einer Reihenfolge von einzuhüllenden Komponenten, Eingaben etc., erfassen kann und gleichzeitig mögliche Fehleingaben, beispielsweise bzgl. der Steuerung einer Bildmessung oder der Protokollplanung oder einer Positionsplanung für eine Bohrschablone, vorteilhaft ignoriert.

In einer ebenfalls bevorzugten Ausführungsvariante wird bei Aktivierung des Hüll-Modus auf einer Ausgabeeinheit, insbesondere der Ausgabeeinheit der Bildgebungsanlage für den Nutzer eine Anzeige von Instruktionen zum Bedecken einer äußeren Gehäusefläche wenigstens einer Komponente der Anlage, ein Verfahrensfortschritt und/oder das Ergebnis eines Prüfschrittes für die Startposition, eine Zwischenposition und/oder die Endposition gestartet. Instruktionen für den Nutzer können beispielsweise sein: Reihenfolge verschiedener Komponenten, spezielle Anordnungen, Handgriffe bzw. Korrekturbewegungen für einzelne Hüllelemente und/oder Komponenten, eine Verstell-Schrittfolge für einzelne Komponenten oder Ausgangsposition des Hüllelements für wenigstens eine Komponente, Hinweise zum sachgemäßen Verbinden von zwei Hüllelementen, etc. Durch die Anzeige wird ein Nutzer vorteilhaft durch das erfindungsgemäße Verfahren geführt. Er hat beispielsweise auch die Möglichkeit, über entsprechende Eingabe eine Schrittfolge zum Bedecken wenigstens einer Komponente virtuell zu durchlaufen. Über entsprechende Eingabe kann der Nutzer die Information, die er benötigt, zur Anzeige bringen. Anderseits wird der Nutzer beim Bedecken einer oder mehrerer Komponenten instruktiv begleitet. Dies vermindert Fehler, spart Zeit und ermöglicht es auch, weniger erfahrenem Personal, den Einhüll-Prozess allein durchzuführen. Die Anzeige kann mittels Piktogrammen, Diagrammen, Video-Clips, Animationen, oder in Textform oder per Sprachausgabe oder dergleichen realisiert sein.

Die Erfindung betrifft in einem weiteren Aspekt eine Recheneinheit zum Bedecken einer äußeren Gehäusefläche wenigstens einer Komponente einer Anlage mit einem sterilen Hüllelement, die Mittel zum Durchführen des erfindungsgemäßen Verfahrens aufweist. Vorteilhaft ist die Recheneinheit in die Anlage integriert. Alternativ kann die Recheneinheit auch entfernt angeordnet sein. Die Recheneinheit kann ausgebildet sein, das gesamte erfindungsgemäße Verfahren umfassend alle Verfahrensschritte, für eine Anlage oder für eine Vielzahl von Anlagen durchzuführen, z.B. in einem Radiologie-Zentrum oder Krankenhaus umfassend beispielsweise mehrere verschiedene Bildgebungsanlagen.

Die Erfindung betrifft in einem weiteren Aspekt ein Computerprogramm mit Programmcode, um das erfindungsgemäße Verfahren durchzuführen, wenn das Computerprogramm auf einem Computer ausgeführt wird.

Die Erfindung betrifft in einem letzten Aspekt auch einen computerlesbaren Datenträger mit Programmcode eines Computerprogramms, um das erfindungsgemäße Verfahren durchzuführen, wenn das Computerprogramm auf einem Computer ausgeführt wird. Vorteilhaft können die Verfahrensschritte auf einem Computer, beispielsweise in einer Recheneinheit einer Anlage, insbesondere einer medizinischen Bildgebungsanlage, ausgeführt werden.

Die oben beschriebenen Eigenschaften, Merkmale und Vorteile dieser Erfindung sowie die Art und Weise, wie diese erreicht werden, werden klarer und deutlicher verständlich im Zusammenhang mit der folgenden Beschreibung der Ausführungsbeispiele, die im Zusammenhang mit den Zeichnungen näher erläutert werden. Durch diese Beschreibung erfolgt keine Beschränkung der Erfindung auf diese Ausführungsbeispiele. In verschiedenen Figuren sind gleiche Komponenten mit identischen Bezugszeichen versehen. Die Figuren sind in der Regel nicht maßstäblich. Es zeigen:
- FIG 1: eine Ansicht einer Anlage in Form einer medizinischen Bildgebungsanlage, ausgebildet als C-BogenRöntgengerät gemäß einem Ausführungsbeispiel der vorliegenden Erfindung,
- FIG 2: ein schematisches Ablaufdiagramm eines erfindungsgemäßen Verfahrens in einem Ausführungsbeispiel,
- FIG 3: einen Anzeigemonitor einer Anlage, insbesondere einer Bildgebungsanlage, mit einer Veranschaulichung entsprechend einem ersten Verfahrenszustand gemäß einem Ausführungsbeispiel der Erfindung,
- FIG 4: einen Anzeigemonitor der Anlage mit einer Veranschaulichung entsprechend einem zweiten Verfahrenszustand gemäß einem Ausführungsbeispiel der Erfindung, und
- FIG: 5 einen Anzeigemonitor der Anlage mit einer Veranschaulichung entsprechend einem weiteren Verfahrenszustand gemäß einem Ausführungsbeispiel der Erfindung.

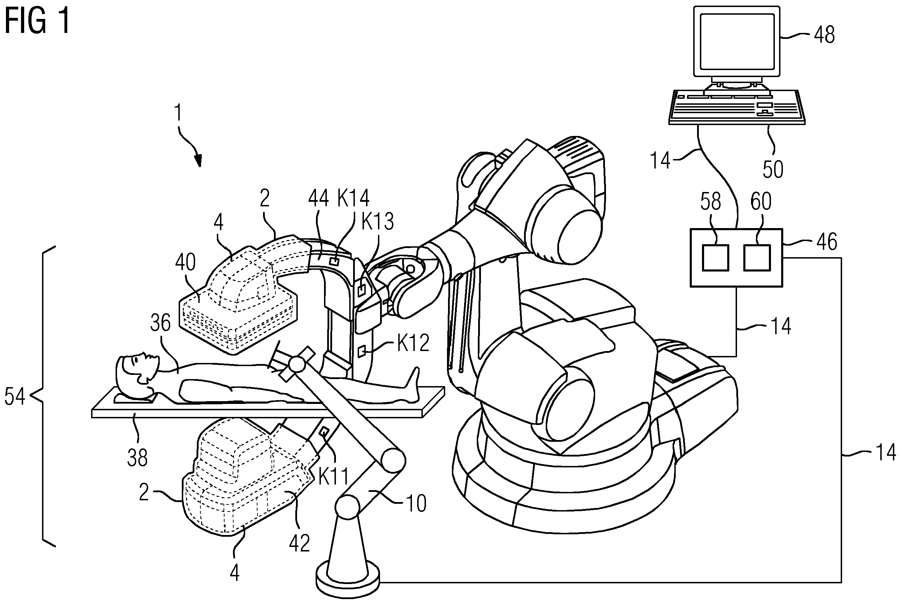

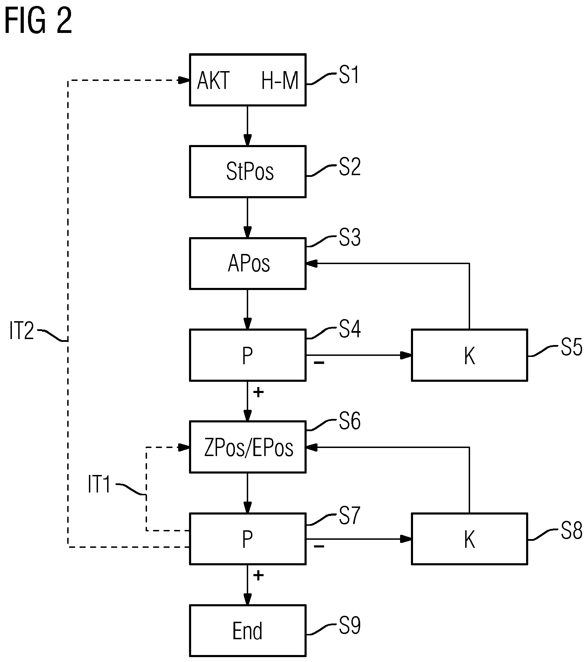

**Figur 1** zeigt eine erfindungsgemäße Anlage in Form einer medizinische Bildgebungsanlage 1 in einem Ausführungsbeispiel. Hier handelt es sich um ein monoplanes angiographisches Röntgensystem 1 mit einem Ständer in Form eines sechsachsigen Knickarmroboters. Das Röntgensystem 1 umfasst eine Vielzahl von Komponenten 44, 42, 40, 38. Beispielsweise wird eine Komponente in Form eines C-Bogens 44 von dem Ständer gehalten. An den Enden des C-Bogens sind weitere Komponenten in Form einer Röntgenstrahlungsquelle, beispielsweise ein Röntgenstrahler 42 mit Röntgenröhre und Kollimator, und ein Röntgenbilddetektor bzw. Röntgendetektor 40 als Aufnahmeeinheit 54 angebracht. Das erfindungsgemäße angiographische Röntgensystem 1 ist insbesondere um Drehzentren und Drehachsen in der C-Bogen-Ebene des Röntgenbilddetektors 40 drehbar. Im Strahlengang des Röntgenstrahlers 42 befindet sich auf einer Patientenliege 38 ein zu untersuchender Patient 36 als Untersuchungsobjekt. Die Komponenten Röntgenstrahler 42 und Röntgendetektor 40 weisen jeweils eine äußere Gehäusefläche 4 auf, die bereits jeweils durch ein Hüllelement 2 in Form von sterilen Folien 2 beispielhaft bedeckt bzw. umhüllt wird. Die Folien 2 sind mittels erfindungsgemäßem Verfahren zum Bedecken einer äußeren Gehäusefläche angebracht worden, wie weiter unten mit Bezug zu Figur 2 näher erläutert wird. Auch die Komponente C-Bogen 44 kann erfindungsgemäß eingehüllt werden (dann jedoch in der Regel als erste, zu bedeckende Komponente einer Mehrzahl von Komponenten). Zu diesem Zweck umfasst der C-Bogen 44 eine Vielzahl von Kontaktelementen K11, K12, K13 und K14, die in seinen seitlichen Gehäuseaußenflächen angeordnet sind und einen Abstand von 40cm aufweisen. Diese Kontaktelemente können beispielsweise als Reed-Kontaktelemente oder als RFID-Kontaktelemente ausgebildet sein. Jedes Kontaktelement repräsentiert eine aus einer Vielzahl von für den Einhüll-Prozess vordefinierten Zwischenpositionen und dienen der Überprüfung, ob das Hüllelement 2 in der betrachteten Zwischenposition ordnungsgemäß entsprechend der Zwischenposition angeordnet ist. Zu diesem Zweck umfasst auch das Hüllelement entsprechende Kontaktelemente (nicht dargestellt), wobei jeweils ein Kontaktelement von Hüllelement 2 und C-Bogen 44 ein Kontakt-Paar bezüglich einer der vordefinierten Zwischenpositionen bilden. Eine weitere Anlage kann durch eine Robotereinheit in Form eines Schablonen- bzw. Führungsroboters 10 gebildet werden. Dieser befindet sich während eines medizinischen Eingriffs so wie die Bildgebungsanlage 1 auch dicht am Patienten 36 und sollte daher ebenfalls zumindest weitgehend steril abgedeckt sein. Der Führungsroboter 10 dient zur Unterstützung und/oder Führung von Bewegungen des operierenden Arztes. Der Roboter 10 kann insbesondere um mehrere Achsen und Gelenke verschwenk-, verdreh- und/oder verkippbar ausgebildet sein, da die Position der von ihm in einer festen Relativlage zur Anatomie des Patienten 36 fixierten Führungsinstrumente beliebig einstellbar sein muss. Der Führungsroboter 10 kann zum Datenaustausch mit der Steuereinheit 46 der Bildgebungsanlage in bekannter Weise kabellos oder kabelgebunden über Verbindung 14 verbunden sein. Über diese Verbindung 14 kann der Roboter 10 beispielsweise angesteuert werden und/oder Koordinaten für seine Führungsinstrumente empfangen.

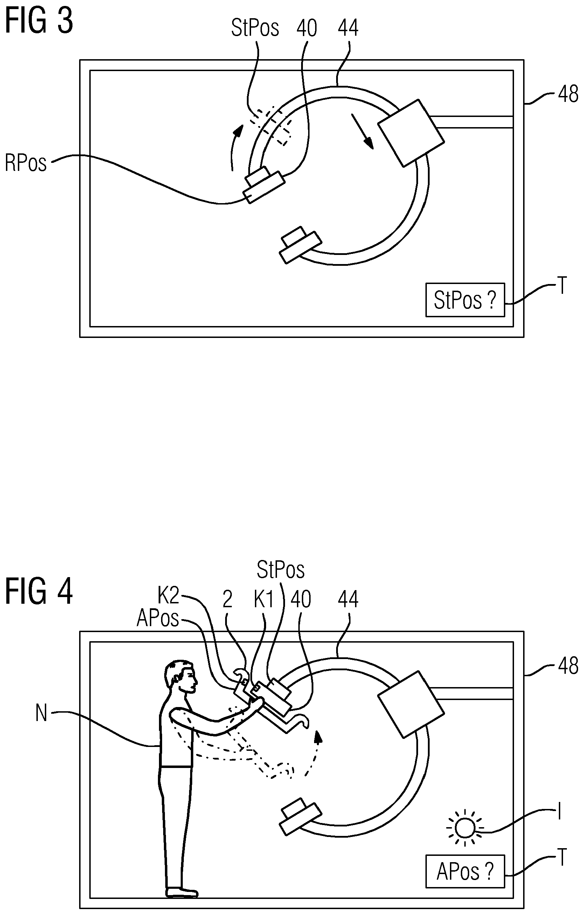

Die Steuereinheit 46 kann als eigenständige Steuereinheit oder als Teil einer Steuereinheit des angiographischen Röntgensystems 1 ausgebildet sein. Die Steuereinheit 46 steht mit einer Ausgabeeinheit 48 und einer Eingabeeinheit 50 über eine weitere Verbindung 14 in Datenkommunikation. Die Ausgabeeinheit 48 dient beispielsweise zur graphischen Anzeige von Verfahrenszuständen des erfindungsgemäßen Verfahrens, wie in den Figuren 3 bis 5 näher gezeigt. Die Ausgabeeinheit dient auch zur Anzeige eines Prüfergebnisses im Hinblick auf einen korrekten Sitz des Hüllelements 2. Sie kann aber auch Instruktionen für einen Nutzer N anzeigen, beispielsweise Lehrfilme über das richtige Anlegen eines Hüllelements 2 für eine bestimmte Komponente einer der Anlagen 1, 10. Die Eingabeeinheit 50 dient beispielsweise der Bestätigung des korrekten Sitzes des Hüllelements 2, der Unterbrechung des erfindungsgemäßen Verfahrens zu einem durch den Nutzer N bestimmten Zeitpunkt oder der Auswahl von gewünschter, anzuzeigender Information oder der Auswahl bzw. Bestätigung einer gewünschten Prozessroutine für ein erfindungsgemäßes Verfahren. Bei der Ausgabeeinheit 48 kann es sich beispielsweise um einen LCD-, Plasma- oder OLED-Bildschirm handeln. Es kann sich weiterhin um einen berührungsempfindlichen Bildschirm handeln, welcher auch als Eingabeeinheit 50 ausgebildet ist. Bei der Eingabeeinheit 50 handelt es sich beispielsweise um eine Tastatur, eine Maus, einen sogenannten "Touch-Screen" um ein Mikrofon zur Spracheingabe und/oder einen Fußschalter. Mikrofon und Fußschalter haben insbesondere den Vorteil, dass die Hände des Nutzers N frei bleiben. Die Eingabeeinheit 50 kann auch eingerichtet sein, um Bewegungen eines Benutzers zu erkennen und in entsprechende Befehle zu übersetzen.

Die Steuereinheit 46 steht auch mit der Aufnahmeeinheit 54 zum Datenaustausch in Verbindung 14. Es können zum Beispiel Steuersignale zur Ausgabe von akustischen Warnsignalen oder Steuersignale zum Unterbrechen oder Stoppen einer Bildmessung übertragen werden. Die Datenverbindungen 14 sind jeweils in bekannter Weise kabelgebunden oder kabellos realisiert.

Die Steuereinheit 46 umfasst eine Recheneinheit 58, die dazu eingerichtet ist Steuerbefehle für eine Anlage 1, 10 zu erzeugen, die diese in einem Hüll-Modus H-M derart verstellt, dass eine Komponente 40, 42, 44, 38 der Anlage 1, 10 eine vorab definierte Startposition StPos und/oder Endposition EPos einnimmt. Bevorzugt erzeugt die Recheneinheit 58 auch entsprechende Steuersignale für ggf. vorhandene erfindungsgemäße Zwischenpositionen ZPos (nicht dargestellt) einer der Komponenten. Entsprechend kann die Recheneinheit 58 wenigstens einen Koordinatensatz für eine der KomponentenPositionen, der z.B. in einem von der Steuereinheit 46 ebenfalls umfassten Speicher 60 zum automatischen Abruf hinterlegt ist, abrufen bzw. auslesen, sofern eine durch den Nutzer ausgewählte Prozessroutine für ein erfindungsgemäßes Verfahren das Einstellen dieser Position vorsieht. Die Recheneinheit 58 ist ferner dazu eingerichtet, Signale von an wenigstens einer Komponente 44 angeordneten Kontaktelementen K11 bis K14 im Hinblick auf einem Kontaktschluss zur Erstellung eines Prüfergebnisses im Hinblick auf einen korrekten Sitz des Hüllelements 2 in einer der Positionen zu empfangen und auszuwerten.

Alle genannten Einheiten können auch körperlich oder funktional mit einander verbunden sein.

Die Recheneinheit 58 kann mit einem computerlesbaren Datenträger zusammenwirken, insbesondere um durch ein Computerprogramm mit Programmcode ein erfindungsgemäßes Verfahren durchzuführen. Weiterhin kann das Computerprogramm auf dem maschinenlesbaren Träger abrufbar gespeichert sein. Insbesondere kann es sich bei dem maschinenlesbaren Träger um eine CD, DVD, Blu-Ray Disc, einen Memory-Stick oder eine Festplatte handeln. Die Recheneinheit 58 kann in Form von Hard- oder in Form von Software ausgebildet sein. Beispielsweise ist die Recheneinheit 58 als ein sogenanntes FPGA (Akronym für das englischsprachige "Field Programmable Gate Array") ausgebildet oder umfasst eine arithmetische Logikeinheit.

In dem hier gezeigten Beispiel ist in dem Speicher 60 der Steuereinheit 46 wenigstens ein Computerprogramm gespeichert, welches alle Verfahrensschritte des erfindungsgemäßen Verfahrens durchführt, wenn das Computerprogramm auf dem Computer ausgeführt wird. Das Computerprogramm zur Ausführung der Verfahrensschritte des erfindungsgemäßen Verfahrens umfasst Programmcode. Weiterhin kann das Computerprogramm als ausführbare Datei ausgebildet sein und/oder auf einem anderen Rechensystem. Beispielsweise kann die Anlage 1, 10 so ausgelegt sein, dass die Recheneinheit 58 das Computerprogramm zum Ausführen des erfindungsgemäßen Verfahrens über ein Intranet oder über das Internet in seinen internen Arbeitsspeicher lädt.

**Figur 2** zeigt ein schematisches Ablaufdiagramm eines erfindungsgemäßen Verfahrens in einem Ausführungsbeispiel. Das Verfahren gemäß Ausführungsbeispiel umfasst eine Vielzahl von Schritten S1 bis S8. Diese sind zum Teil optional. Das Verfahren gemäß diesem Ausführungsbeispiel kann zumindest teilweise in Iterationsschleifen IT1, IT2 durchlaufen werden.

In einem ersten Schritt S1 erfolgt eine Aktivierung AKT eines Hüll-Modus H-M einer Anlage 1, 10. Die Aktivierung AKT kann beispielsweise auf Nutzereingabe mittels Ausgabe- und Eingabe-Einheit 48, 50 erfolgen. In diesem Hüll-Modus H-M greift die Anlage 1, 10, insbesondere ihre Recheneinheit 46 auf hinterlegte Routinen bzw. Prozessabläufe zum Bedecken bzw. Verpacken wenigstens einer, bevorzugt mehrerer Komponenten der Anlage 1, 10 zu, die beispielsweise in einem Speicher 60 der Anlage 1, 10 lokal, aber auch zentral hinterlegt sein können. Hinterlegte Routinen können beispielsweise bei einer Konfiguration oder zusammen mit einem Update der Hüll-Modus-Funktion neu eingestellt, angepasst, ergänzt oder erweitert werden. Bei Aktivierung AKT des Hüll-Modus H-M kann der Benutzer in diesem Ausführungsbeispiel auch Eingaben bzgl. einer geplanten Bildmessung bzw. zu einem geplanten operativen Eingriff machen. Entsprechend dieser Angaben kann die Recheneinheit 46 eine oder mehrere passende Routinen auswählen, in welcher jeweils alle Komponenten, die für die Bildmessung bzw. den medizinischen Eingriff steril bedeckt sein müssen und eine Reihenfolge definiert ist, in der die Komponenten bedeckt werden sollen. Verschiedene Routinen können dem Benutzer zur Auswahl angezeigt werden. Entsprechend der gewählten Routine identifiziert die Recheneinheit die wenigstens eine, erste Komponente der Anlage 1, 10, die verpackt werden soll. In diesem Ausführungsbeispiel ist dies der Röntgendetektor 40. Ferner ruft die Recheneinheit 46 die für den Röntgendetektor 40 hinterlegten Koordinaten seiner Startposition StPos im Bezug zum Koordinatensystem der Bildgebungsanlage 1 ab. Der Röntgendetektor 40 steht in diesem Ausführungsbeispiel repräsentativ für jede beliebige andere Komponente der Anlage 1, 10 insbesondere auch für eine Komponente der Roboter-Einheit 10.

Aus Sicherheitsgründen wird auf Aktivierung AKT des HüllModus H-M der Steuermechanismus der Anlage in Form der Bildgebungsanlage 1 derart blockiert, dass keine Bildmessung mehr erfolgen kann. Lediglich Eingaben des Nutzers, die für ein erfindungsgemäßes Bedecken wenigstens einer Komponente dienen, werden ausgewertet und verarbeitet.

In einem zweiten Verfahrensschritt S2 wird die Anlage 1, 10 derart verstellt, dass der Röntgendetektor 40 in seine Startposition StPos gebracht wird. In diesem Fall kann dies eine Bewegung des Roboterarm-Ständers der Anlage 1, 10 einschließen, je nach Ruheposition der Anlage 1, 10, oder sich auf eine Verschiebung des C-Bogens 44 durch einen optional vorhandenen Schlitten beschränken. Die Startposition StPos ist dadurch gekennzeichnet, dass sie für den Nutzer händisch gut erreichbar ist. Insbesondere ermöglicht sie, dass der Nutzer sie in einer aufrechten, also bequemen Haltung erreichen kann.

Parallel zu den Schritten S1 und S2 kann dem Nutzer im aktivierten Hüll-Modus H-M eine Veranschaulichung bzw. Darstellung zur Anzeige auf dem Anzeigemonitor 48 ausgegeben werden. Ein Anzeigemonitor 48 einer Anlage 1, 10 zu diesem ersten Verfahrenszustand ist in **Figur 3** gezeigt. Die Veranschaulichung kann durch eine vereinfachte Abbildung, ein Piktogramm, der Anlage 1, 10, hier der Bildgebungsanlage 1, gebildet sein, bei welcher beispielsweise durch Pfeile angedeutet, wird, wo sich die Ruheposition RPos und die Startposition StPos des Röntgendetektors 40 oder einer beliebigen anderen, zu bedeckenden Komponente befindet und/oder welche Bewegung die Anlage 1, 10 ausführen muss, damit der Röntgendetektor 40 die Startposition StPos erreicht. Anstelle eines unbeweglichen Piktogramms kann auch ein instruktiver Video-Clip, ebenfalls bevorzugt Piktogramm-artig die genannten Positionen bzw. Bewegungsabläufe veranschaulichen.

Der Hüll-Modus H-M dieses Ausführungsbeispiels ist interaktiv. Mit anderen Worten beachtet bzw. erwartet bzw. fordert die Recheneinheit 46 der Anlage 1, 10 Nutzer-Input. Darum umfasst die in Figur 3 gezeigte Veranschaulichung auch ein Textfeld T, indem der Nutzer gefragt wird, ob der Röntgendetektor 40 die Startposition StPos eingenommen hat. Bestätigt der Nutzer, beispielsweise per Spracheingabe die Startposition StPos für den Röntgendetektor 40, führt die Pozess-Routine das Verfahren bzw. den Nutzer zum folgenden Verfahrensschritt. Ohne die bestätigende Eingabe des Nutzers kann der folgende Verfahrensablauf gesperrt sein.

In einem weiteren Schritt S3 erfolgt das Positionieren des Hüllelements 2 in seiner Ausgangsposition APos. Die Ausgangsposition APos ist bevorzugt nahe, also beispielsweise nur wenige Zentimeter, max. 10cm, von der Startposition StPos entfernt. Die Positionierung erfolgt bevorzugt durch den Nutzer. Dieser hält bzw. umgreift das Hüllelement 2 derart, dass die Sterilität der Außenseite des Hüllelements 2 erhalten bleibt. Das Positionieren des Hüllelements 2 in der Ausgangsposition APos umfasst auch ein unter Umständen notwendige Auffalten, Aufrollen, Aufklappen und/oder Öffnen des Hüllelements 2. Dies ist Abhängig von der jeweiligen Form und/oder Größe des Hüllelements 2. In dieser Ausführung ist das Hüllelement tüten-förmig ausgebildet, mit anderen Worten ist es in fünf von sechs Rumrichtungen geschlossen. Hier wird das tütenförmige Hüllelement 2 zwischen Öffnung und gegenüberliegendem Boden gestaucht und so in die Ausgangsposition APos verbracht, sodass die Bodeninnenseite im Wesentlichen der Detektorinnenfläche gegenüber liegend angeordnet ist. Die Ausgangslage und das ggf. notwendige Auffalten, Aufrollen, Aufklappen und/oder Öffnen des Hüllelements 2 kann für beliebige andere Komponenten anders ausgestaltet sein und ergibt sich im Wesentlichen in Abhängigkeit von Größe und/oder Form der Komponente. Schritt S3 kann auch umfassen, das Hüllelement 2 in der Ausgangsposition APos mittels Befestigungsmitteln wie Klemmen oder Klebestreifen zu fixieren, sodass der Nutzer das Hüllelement 2 nicht während des gesamten zweiten Verstellens in der Ausgangsposition APos halten muss.

In einem Folgeschritt S4 überprüft P die Anlage 1, 10 für den Röntgendetektor 40 in der Startposition StPos den korrekten Sitz des Hüllelements 2. Dazu erfasst die Anlage 1, 10, ob ein Kontaktschluss zwischen einem seitlich an der Detektoraußenfläche und seitlich kurz über dem Boden des Hüllelements 2 angeordneten Kontaktelementen K1, K2 vorliegt oder nicht. Alternativ kann der Nutzer mittels Sichtkontrolle den korrekten Sitz des Hüllelements 2 in der Startposition StPos verifizieren. Ist das Hüllelement 2 noch nicht sachgemäß positioniert (-), kann der Nutzer N in einem Korrekturschritt S5 die Position des Hüllelements 2 händisch anpassen (K), indem er das Hüllelement 2 leicht in Richtung der Ausgangsposition APos verschiebt. Auch hier kann ohne positives Prüfergebnis bzw. entsprechende Bestätigung durch den Nutzer die Prozessroutine zur Sicherheit abgebrochen werden.

Parallel zu den Schritten S3, S4 und S5 kann dem Nutzer im aktivierten Hüll-Modus H-M eine Veranschaulichung bzw. Darstellung zur Anzeige auf dem Anzeigemonitor 48 ausgegeben werden. Ein Anzeigemonitor 48 einer Anlage 1, 10 zu diesem zweiten Verfahrenszustand ist in **Figur 4** gezeigt. Auch hier können ähnlich gestaltete Piktogramme und/oder Clips eingesetzt werden, um dem Nutzer N zu veranschaulichen, wie das Hüllelememt 2 in die Ausgangsposition APos verbracht bzw. gehalten werden muss, beispielsweise wieder durch Pfeile. Die in Figur 4 gezeigte Veranschaulichung umfasst auch ein Textfeld T, indem der Nutzer gefragt wird, ob das Hüllelement 2 die Ausgangsposition APos eingenommen hat. Bestätigt der Nutzer, beispielsweise per Spracheingabe die Ausgangsposition APos für das Hüllelement 2, führt die Prozess-Routine das Verfahren bzw. den Nutzer zum folgenden Verfahrensschritt. Ohne die bestätigende Eingabe des Nutzers N kann der folgende Verfahrensablauf gesperrt sein. Die Darstellung kann optional ein zusätzliches Feld, beispielsweise ein Textfeld, umfassen (nicht dargestellt), welches angibt, welches Hüllelement bestmöglich für die aktuell zu bedeckende Komponente 40 zu verwenden ist, oder welche Alternativen es gibt. Zudem kann angegeben sein, wie das Hüllelement 2 optimal geöffnet, gefaltet, aufgerollt oder dergleichen wird. Die Darstellung kann zudem ein Indikator-Feld I umfassen, welches dem Nutzer N optisch Aufschluss über ein Ergebnis einer automatischen Prüfung in Hinblick auf einen Kontaktschluss und damit den sachgemäßen Sitz und die korrekte Anordnung des Hüllelements 2 in der Ausgangsposition APos gibt. Dieses Indikator-Feld I kann bei positivem Prüfergebnis beispielsweise aufblinken oder das Prüfergebnis farblich kodieren. Alternative Ausgabe des Prüfergebnisses ist ebenfalls möglich. In Figur 4 ist beispielhaft auch die Positionierung der Kontaktelemente K1 und K2 beispielshaft veranschaulicht.

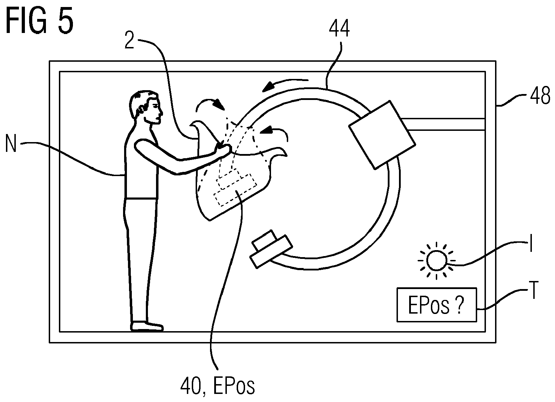

Auf die bestätigende Eingabe des Nutzers N, wird Schritt S6 (Prüfschritt positiv, +) gestartet. Dieser umfasst ein zweites Verstellen der Anlage 1, 10 derart, dass sich der Röntgenbilddetektor 40 in das durch den Nutzer N gehaltene Hüllelement 2 hinein bewegt bzw. verfährt. Durch diese Verstellbewegung erst wird der Röntgenbilddetektor 40 im Wesentlichen vollständig durch das Hüllelement 2 bedeckt bzw. eingeschlossen. Die Verstellbewegung ist vorliegend eine kontinuierliche Bewegung (veranschaulicht in **Figur 5** durch einen gestrichelt linierten Pfeil entlang des C-Bogens 44), also unterbrechungsfrei bis zum Erreichen einer Endposition Epos des Röntgendetektors 40. Die Bewegung des Röntgendetektors 40 erfolgt hier entlang eines durch den Radius des C-Bogens 44 definierten Kreisbogen-Abschnitts. Während dieser Verstellbewegung, die mit kontinuierlicher, jedoch langsamer Geschwindigkeit erfolgt, hat der Nutzer die Möglichkeit, das Hüllelement abzurollen, zu entfalten oder zu schließen (wie durch die oberhalb des Hüllelements 2 zum C-Bogen 44 gerichteten Pfeile in Figur 5 angedeutet).

In einem Schritt S7 überprüft P die Anlage 1, 10 für den Röntgendetektor 40 in der Endposition EPos erneut den korrekten Sitz des Hüllelements 2. Dazu erfasst die Anlage 1, 10 ob ein Kontaktschluss zwischen einem weiteren, geeignet platzierten Kontaktelemente-Paar (nicht dargestellt) vorliegt oder nicht. Alternativ kann der Nutzer wieder mittels Sichtkontrolle den korrekten Sitz des Hüllelements 2 in der Endposition EPos verifizieren. Ist das Hüllelement 2 noch nicht sachgemäß positioniert (-), kann der Nutzer N in einem Korrekturschritt S8 die Position des Hüllelements 2 händisch anpassen (K), indem er das Hüllelement 2 leicht verändert, solange, bis der Prüfschritt S7 einen Kontaktschluss ergibt bzw. der Nutzer die Position für sachgerecht hält. Auch hier kann ohne positives Prüfergebnis bzw. entsprechende Bestätigung durch den Nutzer die Prozessroutine zur Sicherheit abgebrochen werden.

Parallel zu den Schritten S6, S7 und S8 kann dem Nutzer im aktivierten Hüll-Modus H-M eine Veranschaulichung bzw. Darstellung zur Anzeige auf dem Anzeigemonitor 48 ausgegeben werden. Ein Anzeigemonitor 48 einer Anlage 1, 10 zu diesem weiteren Verfahrenszustand ist in **Figur 5** gezeigt. Auch hier können ähnlich gestaltete Piktogramme und/oder Clips eingesetzt werden, um dem Nutzer N zu veranschaulichen, wie der Röntgendetektor 40 in seine Endposition EPos verbracht und ggf. das Hüllelement 2 geschlossen werden muss. Die in Figur 5 gezeigte Veranschaulichung umfasst wieder ein Textfeld T, indem der Nutzer gefragt wird, ob das Hüllelement 2 korrekt am Röntgendetektor 40 in der Endposition EPos sitzt. Bestätigt der Nutzer dies (+), führt die Prozess-Routine das Verfahren bzw. den Nutzer zum Ende des Verfahrens (S9), welches ebenfalls auf dem Anzeigemonitor dargestellt werden kann (nicht gezeigt). Ohne die bestätigende Eingabe des Nutzers N kann das Verfahrensende gesperrt sein. Die Darstellung kann wieder ein Indikator-Feld I umfassen, welches dem Nutzer N optisch Aufschluss über ein Ergebnis einer automatischen Prüfung in Hinblick auf einen Kontaktschluss und damit den sachgemäßen Sitz und die korrekte Anordnung des Hüllelements 2 am Röntgendetektor in der Endposition EPos gibt.

Alternativ zur Endposition EPos des Röntgendetektors 40 kann in Schritt S6 eine Zwischenposition des Röntgendetektors 40 angefahren werden. Diese liegt auf dem Verstellweg des Detektors 40 zwischen Start- und Endposition StPos und EPos. Es können mehrere Zwischenpositionen vorgesehen sein, bei denen die Verstellbewegung automatisch stoppt. Dies Ermöglicht dem Nutzer ein Nachjustieren bzw. Korrigieren der aktuellen Anordnung des Hüllelements 2, sofern erforderlich. Insofern umfasst ein erfindungsgemäßes Verfahren eine Iterationsschleife IT1, die die Schritte S6 bis S8 einschließt und die sich lediglich in der Komponentenposition unterscheiden.

Eine weitere, alternative oder zusätzliche Iterationsschleife IT2 umfasst alle bisherigen Verfahrensschritte S1 bis S8. Sie wird dann durchlaufen, wenn mehrere Komponenten der Anlage 1, 10 nacheinander bedeckt werden müssen, insbesondere umfasst die Iterationsschleife auch ein Verfahren, bei dem sowohl wenigstens eine Komponente der Bildgebungsanlage 1 als auch der Robotereinheit 10 oder lediglich Komponenten der Robotereinheit 10 bedeckt werden müssen.

Es sei nochmals erwähnt, dass der Nutzer N jederzeit eine erfindungsgemäße, zweite Verstellbewegung stoppen und wieder starten kann, beispielsweise per Fußschalter und beispielsweise dann wenn das Hüllelement 2 durch die Verstellbewegung beschädigt zu werden droht, auch wenn sich der Röntgendetektor 40 nicht in einer der vorab definierten, erfindungsgemäßen Komponentenpositionen befindet.

Zusammengefasst ermöglicht die vorliegende Erfindung folgende Vorgehensweisen für das Bedecken verschiedener AnlagenKomponenten, bei der die Anlage bzw. eine ihrer Komponenten zunächst in eine für das Drapen bzw. Einhüllen oder Bedecken komfortable Stellung, die Startposition, gefahren wird.Danach sind drei verschiedene Varianten für das erfindungsgemäße Verfahren denkbar.
1. Aus der Startposition erfolgt eine Bewegung in eine Drape- bzw. Endposition, z.B. mittels einer Nutzerinteraktion (Button),
2. Aus der Startposition erfolgt eine Drape-Bewegung, beispielsweise führt die Robotereinheit eine Bewegung entlang des Verstellwegs durch und der Nutzer hilft der Robotereinheit, in das Hüllelement zu schlüpfen, und/oder
3. Aus der Startposition, erfolgt eine autonome Bewegung der Anlage in das Hüllelement, wobei der Nutzer das Hüllelement beispielsweise an eine Halterung klemmt und die Anlage automatisch in das Hüllelement fährt.

Wo noch nicht explizit geschehen, jedoch sinnvoll und im Sinne der Erfindung, können einzelne Ausführungsbeispiele, einzelne ihrer Teilaspekte oder Merkmale mit einander kombiniert bzw. ausgetauscht werden, ohne den Rahmen der hiesigen Erfindung zu verlassen. Mit Bezug zu einem Ausführungsbeispiel beschriebene Vorteile der Erfindung treffen ohne explizite Nennung, wo übertragbar, auch auf andere Ausführungsbeispiele zu.

## Patentansprüche

1. Verfahren zum Bedecken einer äußeren Gehäusefläche (4) wenigstens einer Komponente (38, 40, 42, 44) einer Anlage (1, 10) mit einem sterilen Hüllelement (2) umfassend die Schritte
- Erstes Verstellen der Anlage derart, dass die wenigstens eine Komponente eine Startposition (StPos) einnimmt (S2),
- Positionieren des sterilen Hüllelements in einer Ausgangsposition (APos) relativ zur Startposition der wenigstens einen Komponente der Anlage (S3),
- Zweites Verstellen der Anlage derart, dass sich die wenigstens eine Komponente der Anlage entlang eines vorab bestimmten Verstellwegs in das Hüllelement hinein bewegt, sodass das Hüllelement die äußere Gehäusefläche der Komponente wenigstens teilweise bedeckt (S6).

2. Verfahren nach Anspruch 1, wobei das zweite Verstellen der Anlage kontinuierlich erfolgt, bis die wenigstens eine Komponente eine vorab definierte Endposition (EPos) eingenommen hat.

3. Verfahren nach Anspruch 1, wobei das zweite Verstellen der Anlage schrittweise zwischen Startposition und einer vorab definierten Zwischenposition (ZPos), zwischen vorab definierten Zwischenpositionen und/oder zwischen vorab definierter Zwischenposition und einer vorab definierten Endposition erfolgt, bis die wenigstens eine Komponente die vorab definierte Endposition eingenommen hat.

4. Verfahren nach einem der vorhergehenden Ansprüche, das auch folgenden Schritt umfasst:
- Prüfen (P) zumindest für die Startposition, eine vorab definierte Zwischenposition und/oder die Endposition der Komponente, ob das Hüllelement die äußere Gehäusefläche der Komponente soweit bedeckt, wie es der jeweiligen Position der Komponente auf ihrem Verstellweg entspricht (S4, S7).

5. Verfahren nach Anspruch 4, wobei das Prüfen umfasst, zu erfassen, ob ein Kontakt zwischen einem die jeweilige Position repräsentierenden ersten Kontaktelement (K1; K11, K12, K13, K14) an der Komponente und einem die jeweilige Position repräsentierenden zweiten Kontaktelement (K2) an dem Hüllelement geschlossen ist.

6. Verfahren nach einem der vorhergehenden Ansprüche, wobei das zweite Verstellen der Anlage auf entsprechende Eingabe durch einen Nutzer (N) hin gestartet und/oder gestoppt werden kann.

7. Verfahren nach einem der vorhergehenden Ansprüche, wobei das erste Verstellen der Anlage auf Aktivierung (AKT) eines Hüll-Modus (H-M) der Anlage hin automatisch erfolgt.

8. Verfahren nach einem der vorhergehenden Ansprüche, wobei bei Aktivierung des Hüll-Modus auf einer Ausgabeeinheit (48) der Anlage für den Nutzer eine Anzeige von Instruktionen zum Bedecken einer äußeren Gehäusefläche wenigstens einer Komponente der Anlage mit sterilem Hüllelement, ein Verfahrensfortschritt und/oder das Ergebnis eines Prüfschrittes für die Startposition, eine Zwischenposition und/oder die Endposition gestartet wird.

9. Verfahren nach Anspruch 8, wobei ein weiteres, zweites Verstellen der Anlage von einer Position in eine entlang des Verstellwegs folgende Position der Komponente nur dann erfolgt, wenn ein vorheriger Prüfschritt ergibt, dass das Hüllelement für eine vorherige Position der Komponente die äußere Gehäusefläche der Komponente soweit bedeckt, wie es der vorherigen Position der Komponente auf ihrem Verstellweg entspricht und/oder der Nutzer dies per Eingabe über eine Eingabeeinheit (50) der Anlage bestätigt.

10. Recheneinheit (58) zum Bedecken einer äußeren Gehäusefläche (4) wenigstens einer Komponente (38, 40, 42, 44) einer Anlage (1, 10) mit einem sterilen Hüllelement (2) aufweisend Mittel zum Durchführen eines Verfahrens gemäß einem der Ansprüche 1 bis 9.

11. Computerprogramm mit Programmcode, um das Verfahren nach einem der Ansprüche 1 bis 9 durchzuführen, wenn das Computerprogramm auf einem Computer ausgeführt wird.

12. Computerlesbarer Datenträger mit Programmcode eines Computerprogramms, um das Verfahren nach einem der Ansprüche 1 bis 9 durchzuführen, wenn das Computerprogramm auf einem Computer ausgeführt wird.

13. Anlage (1, 10) zum Bedecken einer äußeren Gehäusefläche (4) wenigstens einer Komponente (38, 40, 42, 44) der Anlage mit einem sterilen Hüllelement (2) mit einer Recheneinheit (58) nach Anspruch 10.

14. Anlage nach Anspruch 13, wobei die wenigstens eine Komponente und das Hüllelement jeweils wenigstens ein Kontaktelement (K1, K2; K11, K12, K13, K14) umfassen, die derart auf der Gehäuseoberfläche der Komponente bzw. der Oberfläche des Hüllelements angeordnet sind, dass ein Kontaktschluss zwischen denjenigen paarigen, eine jeweilige Position aus Startposition (StPos), vorab definierter, Zwischenposition (ZPos) und/oder Endposition (EPos) der Komponente repräsentierenden Kontaktelementen anzeigt, dass das Hüllelement die äußere Gehäusefläche der Komponente soweit bedeckt, wie es der jeweiligen Position der Komponente auf ihrem Verstellweg entspricht.

15. Anlage nach Anspruch 14, wobei die Kontaktelemente der Komponente und des Hüllelements auf den jeweiligen Oberflächen einen Abstand zwischen 30cm und 70cm aufweisen.
